# EUROPEAN PATENT APPLICATION

(11) **EP 0 545 459 A1**
(43) Date of publication of application: **09.06.1993**
(21) Application number: 92203538.1
(22) Date of filing: 31.01.1990
(51) Int. Cl.: C12Q 1/68, C12N 15/09, C12N 15/11, C12P 19/34

(54) **Amplification of flow-sorted chromosomal DNA**

(30) Priority: 31.01.1989 US 304423
(62) Divisional of application: 90902896.1
(71) Applicant: THE REGENTS OF THE UNIVERSITY OF CALIFORNIA, Oakland, California 94612-3550 (US)
(72) Inventor: Johnson, Daniel Henry, Miami, FL 33129 (US); Gray, Joe W., San Francisco, CA 94116 (US); Tkachuk, Douglas, Menlo Park, CA 94025 (US); Pinkel, Daniel, Walnut Creek, CA 94595 (US)
(74) Representative: Andersen, Henrik Rastrup

(57) **Abstract**

Chromosomal DNA is microdissected to provide segments of known cytological loci and these segments are digested with restriction enzymes. The resulting fragments are ligated to predefined primer duplexes in which only one of the two 5' ends is phosphorylated. The litigation products are amplified by a polymerase chain reaction and the amplified DNA is used to probe cDNA or genomic libraries.

## Description

### BACKGROUND OF THE INVENTION

A "chromosome" is a discrete unit of the genome, carrying many genes. A chromosome is made up of a very long linear molecule of DNA, which, during most of the cell cycle, is complexed with protein.

The somatic cell cycle comprises a long interphase period in which the cell engages in normal metabolic activity, and mitosis, during which the cell divides into two daughter cells. The normal somatic cell exists in the diploid state, that is, it contains paired copies (homologous) of each chromosome. During mitosis, the chromosome pairs separate and replicate to form copies, sometimes called "sister chromatids." Each daughter cell receives one member of each original chromosome pair and one member of each sister chromatid pair.

Germ cells (eggs or sperm) are produced by meiosis. Meiosis divides the germ cell twice and produces four daughter cells, each of which receives a haploid set of chromosomes.

The interphase nuclei of certain organisms (e.g., Drosophila contain giant chromosomes called polytene chromosomes. Polytene chromosomes are produced by the successive replications (up to about 9 times) of a synapsed diploid pair. The replicas do not separate, as in cell division, but rather remain associated. The degree of polyteny is the number of haploid chromosomes contained in the giant chromosome.

Each polytene chromosome presents a series of bands which may be observed under the microscope. The bands form a cytological map of the chromosome. Genetic rearrangements, as seen in a linkage map, can be correlated with structural rearrangements of the cytological map. If a probe for a gene of interest is available, the gene may be localized to a particular band.

The isolation of particular genes is the first step toward a molecular understanding of many biological phenomena. A major impediment to the isolation of many genes of interest is a lack of any knowledge of the gene product. That is, the gene is known only from genetic analysis of a heritable mutant defect (e.g., Huntington's Chorea in man). In these cases the only avenue for gene isolation has been to first define the chromosomal location of the mutant allele by genetic mapping with the hope of finding close linkage to previously cloned DNA. This DNA might then be used to initiate a chromosome walk to the mutant gene locus of interest.

In a "chromosome walk," one begins with a DNA clone thought to lie close to the gene of interest and uses this DNA to isolate homologous DNA clones which extend toward the target gene. If the chromosome walk must extend much more than one hundred kilobases, one almost invariably encounters repetitive DNA sequences which block further chromosome walking toward the target gene.

A chromosome walk of the order of one megabase pairs is considered prohibitive by most molecular biologists due to the abundance of repetitive DNA sequences which can be anticipated to lie between the starting clone and the target gene locus. Unfortunately, a marker clone within one megabase pair of any of several human genes of clinical interest would be considered to be quite close. For example, after several years of intensive work by many laboratories, the gene responsible for Huntington's Chorea is still at least 0.75 megabase pairs distant from the closest DNA marker and closer DNA clones are being sought randomly (F.S. Collins, Symposium Lecture, XVI International Congress of Genetics, Toronto, August 21, 1988). The development of a new method of isolating DNA clones from specific chromosomal regions, which would alleviate most of the problems of chromosome walking, is one of the objects of this invention.

"Microdissection" refers to a technique in which a chromosome is dissected into fragments with a micromanipulator under microscopic observation. "Microcloning" refers to the cloning of the resulting fragments into suitable vectors. Typically, the vectors are then used to transform host cells and thereby create a chromosomal DNA library. The technique has the advantage that clones may be isolated from a cytologically defined chromosomal site. For a more detailed discussion, see Pirotta, Jackle and Edstrom, Genetic Eng. Principles & Methods, 5:1-17 (1983), incorporated by reference herein.

In the past several years a few groups (Johnson, et al., 1987; Dreesen, et al., 1988), have used a microcloning strategy (Scalenghe, et al., 1977; Pirrotta, et al., 1983) to isolate Drosophila genes of interest based on a knowledge of the physical position of the gene in the polytene chromosomes. In this protocol, restriction endonuclease cleavage fragments (usually generated by EcoRI, an endonuclease having a hexameric recognition site) from the microdissected chromosomal DNA are cloned in lambda bacteriophage vectors. Unique DNA fragments in the microcloned bacteriophage library are subsequently used to probe genomic libraries containing much larger chromosomal DNA fragments in order to recover an overlapping series of clones covering the chromosomal region of interest. In this way it was possible to recover 90% of the DNA from a 200 kilobase region of the Drosophila genome containing the APRT gene (Johnson, et al. 1987).

This microcloning strategy has been attempted in organisms lacking polytene chromosomes, such as mouse (Rohme, et al., 1984) and humans with much less success. The major problem here has been the need to dissect many chromosomes to recover a few clones. A major effort to clone DNA from-the human X chromosome fragile site by this procedure failed for this technical reason.

Even with polytene chromosomes, the yield of the desired restriction fragments is small, which reduces the efficiency of the microcloning step and limits the choice of the cloning vector. To maximize efficiency, a phage vector is ordinarily used. However, the packaging systems of phage impose lower and upper limits on the size of the inserted fragment, typically 1-20KB.

It is possible to amplify a specific DNA sequence in a heterogeneous DNA population by means of a polymerase chain reaction (PCR), see Mullis, U.S. 4,683,195; Mullis, U.S. 4,683,202. Conventional PCR requires knowledge of the DNA sequences that flank the DNA sequence to be amplified. This information is used to design primers which hybridize to binding sites lying 3' of the target sequence, one for each of the two strands of the double stranded target DNA. Each primer is hybridized to one of the strands, and the annealed primers are extended across the target sequence by DNA polymerase. The extension products are harvested by denaturation of the duplexes and may now themselves serve as templates for DNA polymerization, as they are also complementary to and capable of binding primers. Thus, each cycle doubles the amount of the DNA synthesized, including the target sequence.

The Mullis PCR technique cannot be used to amplify a target sequence unless the sequence of two flanking regions are known, so that primers may be prepared which hybridize to the flanking regions.

Frohman, et al., PNAS, 85:8998-9002 (1988) describes a modified PCR reaction ("anchored PCR") for amplifying cDNAs. DNAs reversed transcribed from polyadenylated messenger RNA contain a run of thymidines. The cDNA may thus be amplified using two primers: one of which is essentially a homopolymer and the other of which is a known flanking region for the gene of interest. The present method does not require a homopolymeric primer and does not require knowledge of any flanking region. The Frohman method is unsuitable for amplification of genomic DNA as genomic DNA, unlike cDNA, does not generally contain a known homopolymeric sequence.

### SUMMARY OF THE INVENTION

In the present invention, rather than cloning restriction fragments from the very small mass of microdissected chromosomal DNA, such fragments are amplified in vitro using PCR.

In order to permit amplification of the microdissected chromosomal DNA, it is necessary to first fragment the DNA into small (e.g., 100-500 base pairs) fragments in order to produce an efficient substrate for the polymerase chain reaction. In a preferred embodiment, one or both of the restriction endonucleases Rsa-I and Alu-I, which have four base recognition sequences abundant in eukaryotic DNA, are used to digest the DNA. Next it is necessary to ligate an oligonucleotide duplex (preferably about 20-30 base pairs in length) onto these blunt-ended restriction fragments of chromosomal DNA in order to provide a specific primer binding site for the polymerase chain reaction step that follows. Both enzyme digestion and ligations are performed at high efficiency in nanoliter volumes in an oil chamber as described by Pirrotta, et al., 1983. Following the ligation step, the DNA is amplified in 0.1 ml polymerase chain reactions, using one of the oligonucleotides present in the ligated duplex to prime DNA synthesis. Each strand of the oligonucleotide duplex is preferably heteropolymeric, with a 50% GC content being especially preferred.

In theory, 10 femtogram of microdissected DNA (the minimum amount expected to be obtained by microdissection of 10 metaphase chromosomes from vertebrates) could be amplified to yield 100 nanogram of that DNA sequence. Assuming 100 kilobase of sequence to be present in the microdissected DNA, one would expect the amplified product to contain 1 nanogram of DNA per kilobase of sequence.

The amplified sequences may then be cloned, but cloning is not strictly necessary as a large mass of the desired DNA is already available. Even if cloning is undertaken, there is a greater variety of choice of cloning vehicles available as a result of the amplification.

Using the highly efficient random oligonucleotide method and 3000 Ci/mmole ³²P-dATP to label the amplified DNA, a probe could be obtained that would be suitable for screening cDNA libraries for unique complementary DNA sequences. In this way a collection of cDNA clones originating from any particular chromosome region might be obtained. These clones could then be used as probes to isolate an overlapping series of genomic clones from the same chromosomal region. Alternatively, these cDNA clones might be used to search for genetically distinct reStriction-fragment-length-polymorphisms (RFLPs) in genomic Southern blots from individuals where a gene specific defect is expected to be identified in this way. The key element in this "shotgun" approach to cloning DNA from a specific chromosomal region is the recovery of the appropriate cDNA clones. In the preferred strategy proposed here, this will be accomplished by microdissection of the relevant chromosomal region followed by in vitro amplification of the microdissected DNA. The amplified DNA will then be labelled and used as a "shotgun probe" to recover a collection of cDNAs corresponding to the chromosomal region of interest.

### BRIEF DESCRIPTION OF THE DRAWINGS

Figure 1 is a schematic diagram of the overall procedure.

Figure 2 illustrates the effects of uncontrolled phosphorylation of the component oligonucleotides of the primer duplex on the course of the PCR reaction.

Figure 3 illustrates the improvement achieved with controlled phosphorylation as contemplated herein.

### DETAILED DESCRIPTION OF THE PREFERRED EMBODIMENTS

The term "DNA duplex" refers to a double helical complex in which two complementary strands of DNA are held together by hydrogen bonding between complementary bases (Adenosine (A), Thymine (T), Guanine (G), or Cytosine (C)). Each strand of a normal DNA molecule has a 5' phosphate end and a 3' hydroxyl end; these ends may, however, be modified in vitro by chemical or enzymatic means.

Double stranded DNA is cut at specific sites by certain enzymes known as restriction enzymes or restriction endonucleases. The resulting fragments are known individually as "restriction fragments" and collectively as "digests." Many such enzymes are known, and they vary in terms of their recognition and cutting patterns. Some cut both strands at the same point, leaving "blunt ends." Others cut the strands at different points, leaving what are called variously "overhangs," "protruding ends," "cohesive ends," or "sticky ends."

The present invention is not limited to the use of any particular restriction enzyme to obtain chromosomal DNA fragments. Preferably, the enzyme cuts, on average, once every 75 to several thousand bases in the chromosome. Enzymes such as MboI, AluI, HpaII, FnuDII, DpnI, SciNI, HhaI, HaeII, RasI and TaqI, which have four base recognition sites are preferred. Such enzymes would cut random DNA, on average, once every 256 bases. Recognition sites for known restriction enzymes are published annually in the first issue of Nuclei Acids Research by R. J. Roberts. Suppliers include Bethesda Research Labs, Boehringer Mannheim Biochemicals and New England Biolabs.

While cleavage by restriction enzymes is preferred, any other enzymatic, chemical or physical means of achieving the desired degree of fragmentation may be employed, e.g., repeated freeze-thaw cycles. If such other means is used, the ends of the fragments would need to be repaired to be suitable for ligation to the linker duplex of the present invention.

It is desirable to cut the DNA into a larger number of small fragments with an enzyme such as Mbo-I. Short DNA fragments are more efficient substrates for PCR (Saiki, et al., 1988) and the large number of fragments increases the likelihood that ligating linker will be attached at both ends of the chromosomal DNA restriction fragment in order to permit semiconservative replication by PCR.

During replication, the two strands of the double helix separate (a process known as "denaturation"). Each strand then serves as a template for the synthesis of a new strand complementary to the template. That synthesis is directed by enzymes called DNA polymerases.

The synthesis of the complementary strand is facilitated by the presence of a short segment of DNA which is complementary to a portion of the template strand and which is bound thereto. This segment is referred to as a primer; the primer and the corresponding complementary bases on the template strand are referred to collectively as the primer duplex.

The polymerase initiates nucleic acid synthesis at the 3' end of the primer, and proceeds to extend that end along the template strand until polymerization terminates. The ability of a polymerase to continue to synthesize complementary DNA is referred to as its processivity.

The inverse of restriction is ligation. DNA ligases will join a 5'-phosphate end of one DNA molecule to the 3' hydroxyl end of another DNA molecule. They will not, however, join two hydroxyl ends together.

The inverse of denaturation is renaturation (hybridization; annealing). This is the formation of hydrogen bonds between two complementary strands so as to create a DNA complex. Generally speaking, in preparing recombinant DNA by restriction and ligation of DNA from different sources, scientists prefer to use restriction enzymes which leave cohesive ends. A DNA molecule which is blunt-ended can be jointed to any other blunt-ended molecule. One which has a protruding end like that left by MboI, however, can only be ligated to a molecule which has a complementary protruding end, so that the two ends may anneal.

In conventional PCR, sequences flanking the target sequence to be amplified are known and are used as primer binding sites. Complementary oligonucleotides are synthesized and used as primers for the synthesis of "extension products" complementary to the template strand and spanning the target locus.

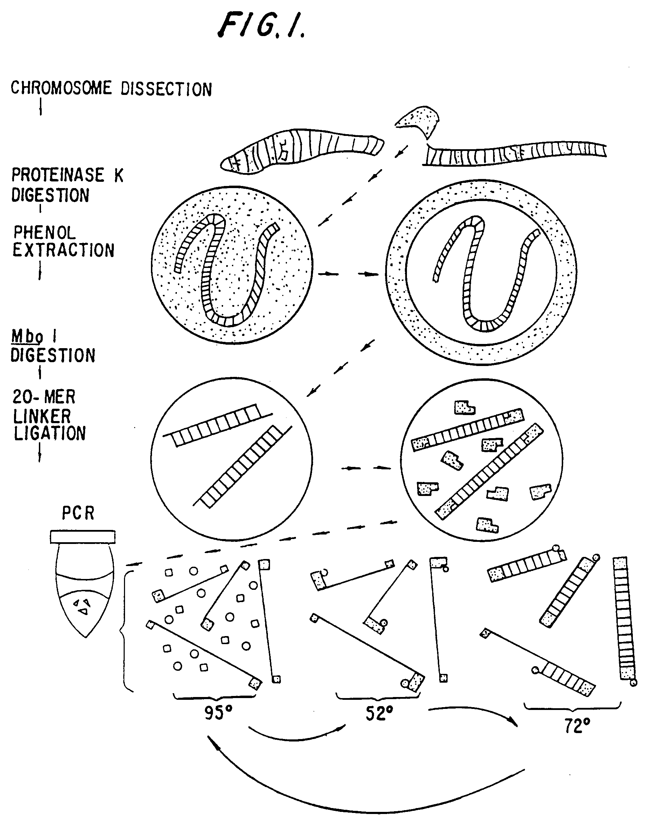

The present invention does not assume any knowledge of the sequences of the chromosomal DNA flanking the target sequence. The primer binding sites are provided by attaching predetermined primer duplexes to the restriction fragments of the chromosomal DNA. The conditions of the ligation reaction are chosen so that essentially every restriction fragment will be ligated to two primer duplexes, forming a "sandwich" as seen in Figure 1. For a theoretical analysis of the effects of DNA concentration and size on ligation, see Dugaiczyk, et al., J. Mol. Biol. (1975) 96, 1.

Preferably, the primer duplex is at least about 20 base pairs. If the duplex is shorter, then the hybrization of the primer to the desired binding site will be less specific and the annealing will be less rapid. If the duplex is longer than about 30 base pairs, the molar concentration of the duplex in the ligation reaction will be reduced so that significantly fewer restriction fragments will be ligated at both ends, as desired, to primer duplexes. Nonetheless, duplexes as long as 150 base pairs could be used in the present invention.

Preferably, the duplex has about 50% GC content, with GC pairs fairly uniformly distributed throughout the duplex.

Preferably, the duplex has a sticky end complementary to the sticky end generated by the aforementioned restriction enzymes. It is permissible, however, to use adaptor DNAs to ligate a restriction fragment with a first sticky end to a primer duplex with a noncomplementary sticky end, or to "blunt end" ligate the restriction fragment with the primer duplex. The primer duplex may also provide an internal recognition site for a different restriction enzyme. This may facilitate later manipulation of the amplified DNA.

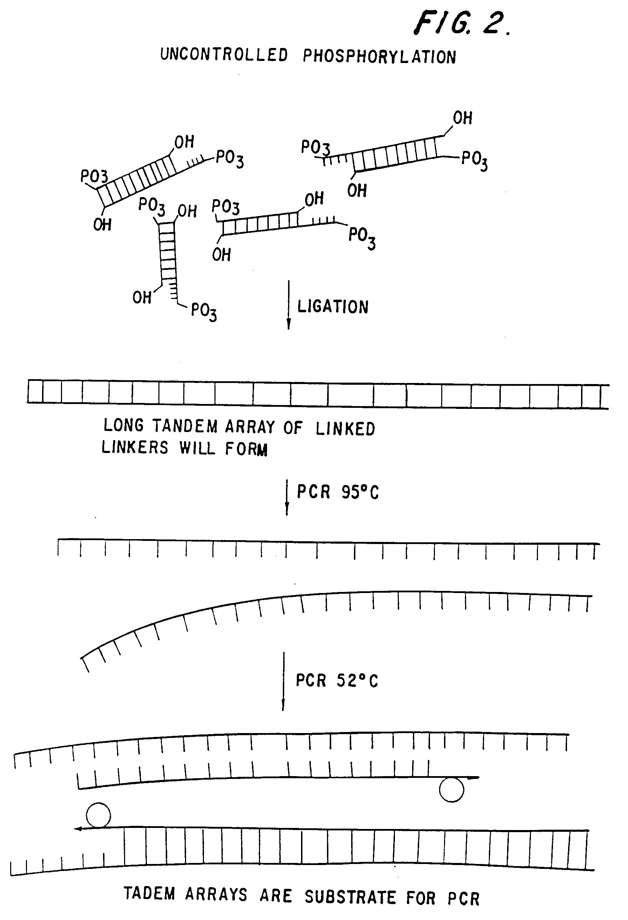

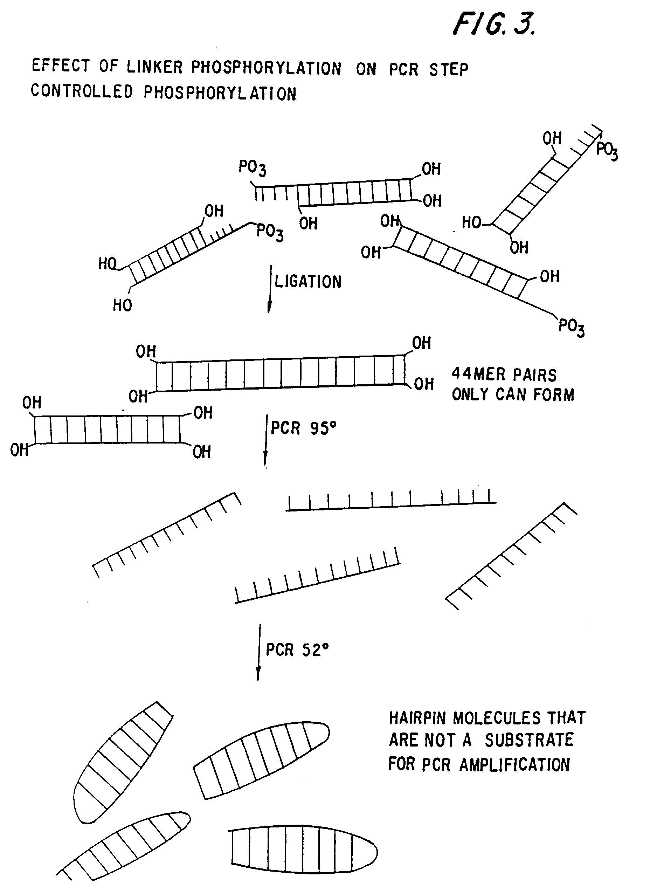

An especially important feature of the present invention is that only one of the two 5' ends of the primer duplex is phosphorylated. The other 5' end is a hydroxyl group as on the two 3' ends. When cohesive ends are ligated, this prevents the formation of long tandem arrays of oligomers during ligation which might then serve as the major substrate in the polymerase chain reaction. The dimer duplex molecules which do form when two linkers ligate together are self-complementary and the hairpin molecules that they form during the annealing step of amplification are not substrates for replication. The difference is shown in Figures 2 and 3.

When the primer duplexes and the fragments are to be ligated by blunt-end ligation, it is desirable that the 5' hydroxyl end of the primer duplex be a protruding end and that the DNA ligase be one which essentially does not ligate single-stranded DNA, e.g., T4 DNA ligase. This is to prevent the formation of long (three or more) tandem arrays of primer duplexes.

DNA ligase catalyzes the formation of a phosphodiester bond between adjacent, 3'-OH and 5'-P termini in DNA. It can be used to ligate both cohesive ends and blunt ends. It follows that even were the primer duplex and the fragment blunt-ended, the primer duplex could be ligated to the fragment in only one orientation, since only one of the 5'-ends of the duplex bears phosphate. In consequence, and in contrast to conventional PCR, only a single primer must be added to the PCR reaction mixture. This primer corresponds to the strand of the primer duplex which bears a 5'OH end; in the example which follows, it is the 20-mer oligonucleotide.

The ligation product of the fragment and the primer duplexes is denatured, yielding two strands. Primer (5' HO-NN ... NN-OH 3') is then permitted to hybridize to each strand. DNA polymerase will extend the 3' end of the primer through the locus of the fragment.

To obtain the desired duplex, each strand is synthesized separately. One strand is treated with a suitable polynucleotide kinase while the other is not (see Maniatis, supra at 396). The two strands are then annealed to form the desired duplex.

After the polymerase chain reaction has run its course, the fragments, all of which are derived from the selected cytological loci, have been amplified. These fragments may be amplified further by cloning them into vectors, using the vectors to transform host cells, and multiplying the host cells. However, one of the advantages of amplification with PCR is that microcloning can be avoided.

There are, nonetheless, advantages to cloning the amplified DNA. The different amplified DNAs differ in utility as probes. Some correspond to sequences which recur frequently in the chromosome; these are of little value. Others are unique and are preferred as probes. It is possible to clone the amplified DNA and screen for individual DNAs which are relatively unique.

In one embodiment, the entire genome is labeled by nick translation with radiolabeled nucleotides, and then digested into fragments with restriction enzymes. These fragments are used as probes to screen the amplified DNA. Repetitive sequences will produce a stronger signal. (Crampton, et al., 1981).

It is not necessary that the entire genome be digested. Instead, particular chromosomes may be selected by any art-recognized technique. One such technique is isolation by flow sorting, see Gray, et al. Cold Spring Harbor Symposia on Quantitative Biology, 51:141-157 (1986). A chromosome may also be selected by microdissection. The desired chromosome(s) are then digested into fragments of suitable size.

In another embodiment, one portion of the amplified DNA is cloned, and the balance is retained for use as a probe. The technique is otherwise identical to that described above.

The amplified DNA, or selected component DNAs chosen by the technique described above, may be denatured and labeled for use as hybridization probes for screening genomic DNA, or more frequently cDNA, libraries. Screening of cDNA libraries is preferred because the cDNA transcripts are related to expressed genes. When a gene is expressed, it is first transcribed into mRNA and then the mRNA is translated into polypeptide. The mRNA may be isolated from a cell and used as a template to prepare a single-stranded complementary DNA (cDNA). This in turn is used as a template for preparation of double stranded cDNA, which is cloned into a vector. A cDNA library is a culture of cells transformed with vectors bearing such cDNA inserts, each transcribed from the mRNA produced by a cell of interest. While the cDNA might not correspond to the complete gene, owing to the processivity limitations of the reverse transcriptase used in their preparation, the cDNA related to a gene of interest may, once identified, be used as a probe to identify the complete gene in a genomic DNA library.

The probe may be labeled in any desired manner compatible with the objectives set forth herein, including, but not limited to labeling with radioisotopes, fluorophores, quenchers, enzymes or enzyme substrates. The label may be attached directly or indirectly, covalently or noncovalently, to the DNA. For example, an avidinated enzyme label may be attached to biotinylated DNA, or a sequence-specific DNA binding protein may be bound to the DNA and then itself bound by a specific, labeled antibody. Preferably, the hybridization probes are prepared using random primers. See Feinberg and Vogelstein (1983), Anal., Biochem., 132, 6; Id., 137, 266. This technique labels the probes with ³²P.

Techniques for the identification of the desired clones by means of hybridization probes are described by Maniatis, et al., at 309, et seq.

### Example 1

The 83D-F region at the base of the right arm of chromosome three of Drosophila melanogaster was chosen as a target for the method of this invention. This region contains the Tpl locus which is unique in the Drosophila genome in that either a duplication or heterozygous deletion of Tpl is lethal (Lindsley, et al., 1972). The 83D-F region was dissected as a unit and as a series of three segments extending across 83E-84E from single salivary gland chromosomes. The chromosome fragments were each deposited in a 1 nl droplet of proteinase K-SDS buffer and deproteinated by phenol extraction (Pirrotta, et al., 1983). The DNA aliquots were digested with Mbo-I in high concentration (40 units/ul).

A micromanipulator fitted with fine glass needles is used to scrape sections from chromosomes that are fixed to a glass coverslip. The dissected DNA fragment is placed in a 1 nl droplet of 10 mM Tris-HCl pH 8.0, 10mM NaCl, 0.1% SDS, 500 g/ml proteinase K which is hanging from a silanized coverslip in a paraffin oil filled chamber to prevent evaporation. The droplet is phenol extracted three times followed by chloroform to remove residual phenol. The DNA is digested by addition of one fifth volume of 250 mM Tris-HCl pH 8.0, 50 mM MgCl₂, 250 mM NaCl containing 20 units / l Mbo-I (obtained by special order from Bethesda Research Labs.), and incubation of the oil chamber at 37^{o}C. This enzyme has a 4 bp recognition sequence and consequently cuts the DNA into a large number of small fragments carrying 4 base 5' cohesive (sticky) ends. An equal volume of a 20mer oligonucleotide duplex with a phosphorylated Mbo-I sticky end as shown below: 
5'PO3 - GATCTGTACTGCACCAGCAAATCC-OH3'
3'HO  -     ACATGACGTGGTCGTTTAGG-OH5'
is added to the droplet followed by one fifth volume of 250 mM Tris-HCl pH 8.0, 50 mM MgCl₂, 5mM ATP, 5mM dithiothreitol, 25% polyethylene glycol and T4 ligase at 4.5 units/ l, and the oil chamber is incubated for 16 hours at 20^{o}C. The concentration of the linker duplex is estimated to be at 100ng/ l in the approximately 5 nl ligation reaction.

The droplet containing the DNA with a defined 20-mer oligonucleotide at each end is recovered from the oil chamber by dilution with 3 l of 10 mM Tris-HCl pH 8.0, 1 mM EDTA. The volume is adjusted to 100 l with the addition of 10 l 500 mM Tris-HCl pH 8.3, 500 mM KCl, 25 mM MgCl₂, 2 l of a 10 mM solution of each deoxynucleotide triphosphate (dATP, dCTP, dGTP and dTTP, ultrapure reagent stock solutions, Pharmacia), 1 l of the appropriate 20 mer oligonucleotide (5' HO-GGATTTGCTGGTGCAGTACA-OH 3') which is complementary to the 24-mer strand of the primer duplex, at 200 ng/ l, 1 l of Tag DNA polymerase (5 units, Perkin Elmer Cetus, Norwalk, CT) and 84 l of dH20. This polymerase chain reaction (PCR) is overlaid with paraffin oil and heated to 95^{o}C for 1.5 minutes to melt the DNA duplex. The reaction mixture is then incubated at 52^{o}C for 2 minutes to anneal the primer to the cohesive ends of the chromosomal DNA molecules and then raised to 72^{o}C to permit semiconservative replication of the DNA molecules by Tag DNA polymerase. This cycle is repeated 25-40 times resulting in the synthesis of 100 ng or more of DNA from a starting mass of 0.1 to 1 pg of chromosomal DNA fragments.

It is important that only the Mbo-I generated, 5' protruding end of the oligomer duplex (5' PO₃-GATCTGTACTGCACCAGCAAATCC-OH3') is phosphorylated.

The amplified chromosomal DNA (PCR probe) appears as a smear of DNA fragments in agarose gels ranging in size from a few hundred to a few thousand base pairs. DNA from the dissected 83D-F region of the salivary gland chromosomes of the third instar larvae of Drosophila melanogaster was amplified and then labelled by nick translation with biotin-16-dUTP (Enzo). The biotinylated DNA was hybridized to chromosomes and the hybridization positions revealed by staining with Streptavadin-horseradish-peroxidase complex using 3,3'diaminobenzidine tetrahydrochloride as substrate.

In situ hybridization of the PCR probes to salivary gland chromosomes produced major signals spanning the dissected interval, with much less intense signals elsewhere. The result indicated that the PCR probes are largely composed of unique DNA sequences complementary to the dissected genomic region. Several cosmid clones isolated by complementarity to the complete 83D-F PCR probe were also examined by in situ hybridization. These experiments reveal the presence of repetitive sequences in cosmid DNA clones from the 83D-F interval. The failure of the PCR probe to reveal strong secondary sites of hybridization, in contrast to the cosmid DNA, probably reflects the greater sequence complexity present in the amplified DNA. The PCR probe is representative of perhaps a few hundred kilobases of genomic DNA sequence (Bossy, et al., 1984) while the cosmid clones contain approximately 40 kilobases of genomic sequence.

To further examine the extent to which the amplified DNA is representative of the dissected chromosome region, a Southern blot of the restricted cosmid DNAs was hybridized with the PCR probe. Approximately 10⁵ cospneo clones of Drosophila melanogaster genomic DNA were screened with the 83D-F PCR probe that was labelled by nick-translation with 3000 Ci/mmole [³²P]dATP. From approximately 100 positive clones, eight were picked. Four of the cosmids from one plate were identical. DNA from cosmids was digested with EcoRl and Xba-I, fractionated on a 1% agarose gel and stained with ethidium bromide. The DNA was blot transferred to nitrocellulose and hybridized with the 83D-F PCR probe. The result showed that most of the larger genomic DNA fragments in these restricted cosmid clones are detected by the PCR probe. Therefore, this probe appears to contain much of the DNA sequence present in the dissected chromosome fragment, as suggested by the results of in situ hybridization.

In cases where a chromosomal region contains abundant repetitive DNA sequences it would be desirable to identify a corresponding collection of unique amplified DNA fragments for use as a probe. The PCR probe obtained from the 83D-F interval was digested with Mbo-I and shotgun cloned into a phosphatased, Bam-Hl opened plasmid. Several hundred bacterial colonies carrying these recombinant plasmids were fixed to nitrocellulose filters and hybridized with total genomic DN in order to identify clones containing repeated DNA sequences (Crampton, et al., 1981). These colonies were removed from the plates. The remaining colonies were pooled, grown in mass and the genomic inserts isolated in mass for use as a probe of the cosmid clone library. This probe, like the complete PCR probe, was also capable of identifying DNA clones originating from the 83D-F region. Another strategy for gene identification using this procedure would be to use individual clones or pools of several clones from the amplified DNA library as probe to identify restriction fragment length polymorphisms in appropriate mutants.

Amplified DNA probes from specific chromosomal regions may also be used for the physical mapping of whole genomes and for filling gaps in the clone collections on these projects.

### Example 2

Although this method has been used to obtain DNA from Drosophila melanogaster polytene chromosomes, it should be useful for the isolation of DNA from specific regions of the non-polytene chromosomes of other organisms as well. The following is a protocol for amplification of DNA from human (non-polytene) chromosomes:
1. Approximately 40,000 metaphase human chromosomes of a specific type (chromosome 22) isolated by flow sorting were suspended in 100 microliters of 10 mM Tris-HCl pH=7.5, 1 mM Na-EDTA, 10 mM NaCl, 0.1% sodium dodecylsulphate, 0.25 miligrams/milliliter proteinase K and incubated at 55 degrees C for 1 hour.
2. The DNA solution was extracted once with an equal volume of phenol/chloroform.
3. The DNA solution was extracted once with an equal volume of chloroform.
4. A one tenth volume of 3M sodium acetate was added with 2.5 volumes of ethanol and this mixture was incubated at -20 degrees C for 16 hours.
5. The DNA precipitate was collected by centrifugation at 14,000 xg for 30 minutes in a microcentrifuge to recover a pellet of purified chromosomal DNA.
6. The pellet of DNA was dissolved in 8 microliters of distilled water to which 1 microliter of React 2 buffer (Bethesda Research Labs), 1 microliter of Mbo-I enzyme (20 units/microliter) were added and the mixture was incubated at 37 degrees C for 1 hour.
7. To the 10 microliter DNA digest in (6), 2 microliters of T4 DNA ligase buffer (BRL), 1 microliter of T4 DNA ligase (9 units/microliter,Bohringer/Mannheim), 1 microliter of the oligonucleotide linker-adapter (0.5 microgram of the aforementioned 20 mer oligonucleotide duplex with a phosphylated Mbo I 4 mer sticky end) and 6 microliters of distilled water were added and the mixture was incubated at 19 degrees C for 16 hours.
8. Two microliters of the ligation product (7) was diluted into a 100 microliter polymerase chain reaction cocktail as described in Example 1 and subjected to 20 cycles of amplification.

This procedure results in the in vitro synthesis of a large collection of DNA sequences from the starting chromosomal DNA. The procedure differs from that previously described for polytene chromosomes in that instead of digesting a single polytene chromosome, a plurality of non-polytene chromosomes are cleaved into the desired fragments. Preferably, at least about 1,000 copies (that is, the same order as the degree of polyteny in the polytene chromosome) of the desired chromosome are provided. The greater the number of copies of the chromosome, and the greater the number of copies of the gene or other genetic element of interest on the chromosome to be amplified, the fewer cycles of PCR are necessary to achieve a desired level of amplification. The amplified non-polytene chromosomal fragments were used as a probe in an in situ hybridization experiment ("chromosome painting", as described by Pinkel, et al., PNAS (USA), 85:9138-42 (1988)) which verified that each fragment hybridized to the chromosome from which it was derived. This collection of amplified DNA molecules can be perpetuated either by subsequent cloning of the material into a plasmid or bacteriophage vector or more easily by additional rounds of in vitro amplification.

The amplified chromosomal DNAs, suitably labeled, may be used in genetic screening. For example, flow sorting may be used to isolate human chromosome 21, which is associated with Down's syndrome, and the present invention may be used to amplify fragments of this chromosome. In an assay for Down's syndrome, embryonic DNA is screened using labeled fragments so obtained. Normals will have only two of chromosome 21; the presence of three such chromosomes is a genetic marker for Down's syndrome.

Chronic myelogenous leukemia is marked by the abnormal "Philadelphia chromosome", which arises through translocation of chromosomes 9 and 22. Chromosomes 9 and 22 are separately isolated and their fragments amplified and used to create a first anti-chromosome 9 hybridization probe and a second anti-chromosome 22 hybridization probe. These probes are differently labeled (e.g., fluorophores of different emission wavelengths). The Philadelphia chromosome will be detected by both probes; normal chromosomes 9 and 22 will strongly hybridize only with one probe or the other.

Similarly, genetic analysis for other changes in the number or constitution of the chromosomes is facilitated by use of probes derived from chromosomal DNA fragments amplified according to the present invention.

### References

Collins, S. (1988) Direct sequencing of amplified genomic fragments documents N-ras point mutations. Oncogene Research, in the press.

Dreesen, T. D., Johnson, D. H. and Henikoff, S. (1988) The brown protein of Drosophila is similar to the white protein and to components of active transport complexes. Mol. Cell. Biol., 8, 5206-5215.

Johnson, D. H., Edstrom J.-E., Burnett, J. B. and Friedman, T. B. (1987) Cloning of a Drosophila melanogaster adenine phosphoribosyltransferase structural gene and deduced amino acid sequence of the enzyme. Gene, 59, 77-86.

Pirrotta, V., Jackle, H. and Edstrom, J.-E. (1983) Microcloning and microdissected chromosome fragments. Genet. Eng. Principles Methods, 5, 1-17.

Rohme, D., Fox, H., Herrman, B., Frischauf, A.-M., Edstrom, J.-E., Mains, P., Silver, L.M. and Lehrach, H. (1984) Molecular clones of the mouse t complex derived from microdissected metaphase chromosomes. Cell, 36, 783-788.

Saiki, R. K., et al., (1988) Primer-directed enzymatic amplification of DNA with a thermostable DNA polymerase. Science, 239, 487-491.

Scalenghe, F., Turco, K. E., Edstrom, J.-E., Pirrotta, V. and Melli, M. (1977) Microdissection and cloning from a specific region of Drosophila melanogaster polytene chromosomes. Chromosoma, 82, 205-216.

Lindsley, D. L., et al., (1972), Genetics, 71, 157.

Bossy, B., Hall, L.M.C. and Spierer, P., (1984), EMBO J., 3, 2537.

Crampton, J.M., Davies, K.E. and Knapp, T.F. (1981), Nucleic Acids Res., 9, 3821.

Simon, J. A., Sutton, C.A., Lobel, R.B., Glaser, R.L. and Lis, J.T. (1985), Cell, 40, 805.

## Claims

1. A method of amplifying DNA fragments from a source DNA which is chromosomal DNA, such as one or more copies of a chromosome and/or one or more copies of a chromosomal segment, that has been flow-sorted, which comprises:
(a) providing double-stranded DNA fragments of said source DNA, said fragments having first and second fragment ends and a length making them suitable as substrates for a polymerase chain reaction;
(b) ligating two molecules of a primer DNA duplex to essentially each DNA fragment, one to the first fragment end and the other to the second fragment end of each DNA fragment thereby obtaining hybrid DNA duplexes, wherein said primer DNA duplexes and ligation conditions are selected to essentially exclude formation of tandem arrays of three or more primer DNA duplexes;
(c) denaturing said hybrid DNA duplexes to obtain first and second hybrid DNA strands, each having a 5' strand end and a 3' strand end;
(d) annealing primer molecules to the 3' strand end of each of said first and second hybrid DNA strands, said primer molecules essentially corresponding in sequence to the second strand of the primer DNA duplex; and
(e) extending said annealed primer to obtain an amplified hybrid DNA duplex, and repeating steps (c)-(e) as often as necessary to obtain a desired degree of amplification.

2. The method according to Claim 1 wherein said primer DNA duplexes consist of a first strand having a 5' phosphate end and a 3' hydroxyl end, and a second and essentially complementary strand having a 3' hydroxyl end and a 5' hydroxyl end.

3. A method of preparing a hybridization probe which comprises amplifying DNA fragments according to the method of Claims 1 or 2 and then (f) preparing the hybridization probes by labeling the amplified DNA fragments.

4. The method according to any of Claims 1 to 3 wherein the source DNA is DNA corresponding to at least about 1,000 copies of said chromosome or of said chromosomal segment.

5. The method according to any of Claims 1 to 4 wherein the chromosome or chromosomal segment is human.

6. The method according to Claim 5 wherein said human chromosome or chromosomal segment is in a metaphase.

7. The method according to any of Claims 1 to 6 wherein said human chromosome or chromosomal segment is selected from the group consisting of chromosome 22, chromosome 21, and chromosome 9 or segments thereform.

8. The method according to any of Claims 1 to 7 wherein the fragments are obtained by digesting the source DNA with a site-specific restriction enzyme to obtain a plurality of DNA fragments, preferably wherein the restriction enzyme has a four base recognition site.

9. The method according to Claim 8 wherein the restriction enzyme generates fragments having sticky ends, and the 5' phosphate end of the primer DNA duplex is a protruding end complementary to and annealable with said sticky ends.

10. The method according to any of Claims 1 to 9 wherein the primer DNA duplex comprises at least about 15 base pairs and less than about 30 base pairs and preferably has about 50% GC composition.

11. The method according to any of Claims 3 to 10 wherein the labeling of the amplified DNA fragments is by such exemplary means as radioisotopes, fluorophores, quenchers, enzymes and enzyme substrates.

12. The method according to any of Claims 3 to 11 wherein the amplified DNA fragments are labeled directly or indirectly.

13. The method according to any of Claims 1 to 12 wherein the primer DNA duplex's 5' hydroxyl end protrudes, and the ligation is performed using a DNA ligase, such as T4 DNA ligase, which is essentially incapable of ligating single-stranded DNA, so as to essentially exclude formation of tandem arrays of three or more primer DNA duplexes.

14. The method according to any of Claims 1 to 13 wherein in preparing the primer duplex, the 5' end of the first strand is phosphorylated by T4 kinase, or the first strand is left with a 5' phosphate end by cleavage with an endonuclease.

15. The method of any of Claims 1 to 14, further comprising cloning amplified chromosomal DNA fragments obtained in step (e) and identifying cloned fragments corresponding in DNA sequence to essentially non-repetitive subsequences of the fragmented chromosomal DNA, and preparing hybridization probes essentially only from the identified fragments, wherein the cloned fragments corresponding to essentially non-repetitive subsequences are optionally identified by (i) screening for hybridization with a labeled digest of the genome of the organism from which the flow-sorted chromosome or chromosomal segment is derived, or (ii) by screening for hybridization with labeled amplified chromosomal DNA fragments obtained from a predetermined chromosome or cytologically defined region or a chromosome.

16. Hybridization probes prepared according to any of Claims 3 to 15.

17. A method of chromosome painting comprising in situ hybridization with one or more appropriate hybridization probes prepared according to any of Claims 3 to 15 to subject chromosomal DNA.

18. A method of chromosome painting which comprises preparing a hybridization probe specific for a selected genetic condition associated with a particular DNA sequence by the method of any of Claims 3 to 15, and then hybridizing the probe in situ to subject chromosomal DNA.

19. A method of genetic screening which comprises chromosome painting a subject chromosomal DNA by the method of Claims 17 or 18, and comparing the hybridization pattern of the subject to a reference pattern to determine any differences in the number or constitution of chromosomes.

20. A method of genetic screening according to Claim 19 wherein embryonic DNA is screened for the presence of three chromosome 21s, a genetic marker for Down's syndrome, using a hybridization probe for chromosome 21.

21. A method of genetic screening according to Claim 19 wherein DNA is screened for the presence of a Philadelphia chromosome, a genetic marker for chronic myelogenous leukemia, using hybridization probes for chromosome 9 and for chromosome 22, wherein the probe for chromosome 9 is differently labeled than the probe for chromosome 22 such as by fluorophores of different emission wavelengths.
